(19) Europäisches Patentamt
European Patent Office
Office européen des brevets

(11) Publication number: 0 416 250 A2

# (12) EUROPEAN PATENT APPLICATION

(21) Application number: 90113594.7

(22) Date of filing: 16.07.90

(51) Int. Cl.[5]: C08B 37/08, //A61K47:36, A61L31:00

(30) Priority: 01.08.89 US 388578

(43) Date of publication of application: 13.03.91 Bulletin 91/11

(84) Designated Contracting States: AT BE DE FR GB IT LU NL SE

(71) Applicant: THE RESEARCH FOUNDATION OF STATE UNIVERSITY OF NEW YORK
PO Box 9
Albany, New York 12201(US)

(72) Inventor: Prestwich, Glenn D.
56 Farm Road
Head of the Harbour, New York 11780(US)
Inventor: Kuo, Jing-wen
23 Spencer Road
Boxborough, Massachusetts 01719(US)
Inventor: Swann, David A.
6 Audobon Road
Lexington, Massachusetts 02173(US)

(74) Representative: Boeters, Hans Dietrich, Dr. et al
Boeters & Bauer Bereiteranger 15
W-8000 München 90(DE)

## (54) N-acylurea and O-acylisourea derivatives of hyaluronic acid.

(57) N-Acylureas and O-acylisoureas prepared by the reaction of a carbodiimide or a biscarbodiimide with hyaluronic acid are useful as vitreous replacements, joint cushions or adjuncts in wound healing.

EP 0 416 250 A2

# N-ACYLUREA AND O-ACYLISOUREA DERIVATIVES OF HYALURONIC ACID

## BACKGROUND OF THE INVENTION

### Field of the Invention

The invention relates to N-acylurea and O-acylisourea derivatives of hyaluronic acid or a salt thereof.

### Brief Description of the Prior Art

N-acylureas and O-acylisoureas are known classes of compounds, which may be prepared by the reaction of a carboxylic acid with a carbodiimide; see for example Tetrahedron Report R101, Tetrahedron vol 37, pgs. 248-282 (Pergamon Press Ltd., 1981).

Tengblad in Biochimica et. Biophysica Acta, 578, pgs. 281-289 (1979) reported coupling a partially digested sodium hyaluronate (Mw of 52,000) to a cyanogen bromide activated agarose using a carbodiimide to effect the coupling of the previously mixed reactants. The immobilized hyaluronate was used as an affinity chromatography surface.

Silver et al. in U.S. Patent 4,703,108 describe cross-linking soluble or insoluble collagen with a carbodiimide in the presence of a "hyaluronate", which is not described further. The product is described as a collagen matrix "impregnated" with a compound (the hyaluronate). From the disclosure, it does not appear to suggest a reaction of hyaluronate with the carbodiimide since the hyaluronate remains free for release from the collagen matrix. However, in the U.S. Patent 4,280,954 to Yannas et al, carbodiimides are suggested as coupling agents to cross-link collagen to hyaluronic acid in particular. The resulting insoluble composites are said to be useful as prosthetic devices for implantation in mammals. No examples are given and one is left to speculate about the products and their mode of preparation.

The N-acylureas and the O-acylisoureas of the present invention are useful intermediates in the preparation of pharmaceutically active drug compositions or compositions which will release pharmaceutically active drugs in a controlled manner.

Therapeutic pharmaceutical drugs are almost exclusively administered to mammals in admixture with an appropriate, pharmaceutically acceptable carrier, for release in the intestinal tract, sub-cutaneous, intra-articular and/or bloodstream of the mammal undergoing therapeutic treatment. Scheduled doses are administered in accordance with a time table designed to maintain a minimum blood or tissue concentration of the drug. The disadvantage of this protocol of administration is that concentration levels of the drug are high following initial administration and low at some time after initial administration. It results in a so-called "peaks and valleys" profile of drug concentrations. This is often undesirable, for example in the sub-cutaneous administration of insulin.

Another problem related to the above-described mode of drug adminstration is the systemic dispersion of the drug throughout the mammal's tissues. Ideally, the drug is targeted to a specific site where it is needed, for example to an intra-articular site, without tissue concentrations appearing systemically, for example in the stomach linings.

To obviate the above-described disadvantages associated with the usual administration of therapeutic drugs, alternative methods of introducing and controlling the release of therapeutic drugs have been sought. Effort has focused on the use of polymeric formulations for the controlled release of drugs using a variety of methods, compositions, and areas of application; see for example "Controlled Release Polymeric Formulations", D. R. Paul and F. W. Harris editors, American Chemical Society, Washington, D.C. 1976.

It will be appreciated that a polymer carrier of a drug must be selected with regard to its pharmaceutical acceptability, i.e.; it must be non-toxic in the quantities employed, compatible with the therapeutic drug it is to carry and it may be bioerodible or catabolically removable. Bioerodible carriers or polymers generally rely on the release of the therapeutic drug as the polymer carrier is eroded away by the in-situ environment through physical processes such as dissolution or by chemical processes such as hydrolysis of the polymer backbone or crosslinks, or by enzymatic degradation. When such polymers are used for delivery of pharmacologically active drugs within medical applications, it is essential that the polymers themselves be nontoxic and that they degrade into non-toxic degradation products as the polymer is eroded by the body fluids. However, many synthetic, biodegradable polymers upon erosion in-vivo yield oligomers and monomers which often adversely interact with the surrounding tissue [D. F. Williams, J. Mater. Sci. 17:1233

(1982)]. In order to minimize the toxicity of the intact polymer carrier and its degradation products, polymers have been designed based upon naturally occurring metabolites. Probably the most extensively studied examples of such polymers are the polyesters derived from lactic or glycolic acid [H. Laufman et al., Surg. Gynecol. Obstet. 145:597(1977); D. L. Wise et al., in "Drug Carriers In Biology And Medicine" (G. Gregoriadis ed.), Academic Press, London, 1979, pages 237-270] and polyamides derived from amino acids [D. A. Wood, Int. J. Pharm. 7:1(1980); S. Yolles et al., in "Controlled Release Technologies: Methods, Theory, And Applications"; A. F. Kydonies, ed. C.R.C. Press, Boca Raton, Florida, 1980, pages 1-6]. Unfortunately, many of these bioerodible polymers erode homogeneously (bulk erosion) which results in uncontrolled and unpredictable release of therapeutic drugs by unfavorable release kinetics. The release kinetics are generally only improved if the polymer carrier is hydrophobic enough to erode heterogeneously, that is by surface erosion rather than bulk degradation [J. Heller, in "Medical Applications Of Controlled Release" (R. S. Langer and D. L. Wise, eds.) C.R.C. Press, Boca Raton, Florida, 1985]. Unfortunately, it is difficult to produce hydrophobic polymeric compositions which degrade by surface erosion and provide the desired release kinetics without themselves being allergenic or toxic or which degrade into allergenic or toxic degradation products. Such hydrophobic polymers have remained a sought after goal.

Certain embodiments of the N-acylurea and O-acylisourea compounds and compositions of the present invention represent a modification of the hydrophilic, water-soluble hyaluronic acid molecule. The modification comprises the insertion of hydrophobic regions on the hyaluronic acid molecule, without converting the whole of the molecule to a hydrophobic, water-insoluble entity. In this way, water-solubility is retained but the advantages of hydrophobicity described above are obtained. The low hydrolytic stability of the hyaluronic acid is increased by the addition of hydrophobic regions to the polymer chain. In use, the hydrophobic modified regions bind lipophilic drugs through weak non-bonded interactions, thereby slowing the diffusion of the drug from the site of administration of a water-soluble, modified hyaluronic acid-drug combination. The modified hyaluronic acid remains at the site of administration and is slowly removed by diffusion and degradation by normal enzymatic processes, safely and without generating toxic or undesirable residues.

In other embodiment compounds and compositions of the invention, adjacent molecules of hyaluronic acid are cross-linked, to lower the water-solubility of the product. The lower solubility compositions are particularly useful, because they tend to "erode" heterogeneously as will be discussed more fully hereinafter. This characteristic is at least a partial solution to the uncontrolled and unpredictable release of therapeutic agents discussed above in relation to the prior art vehicles for drug delivery.

The different modifications of the hyaluronic acid molecule can provide a means of covalently linking the molecule to a therapeutic drug, or can enhance a non-covalent binding or interaction between this water-soluble molecule and an anionic, cationic or hydrophobic drug agent.

## Summary Of The Invention

The invention comprises the N-acylurea and O-acylisourea derivatives of hyaluronic acid or a salt thereof and their use as intermediate compositions in the preparation of therapeutic drug compounds and compositions.

Certain embodiment compositions of the invention are useful as biomaterials, i.e.; as vitreous replacements, joint treatments, adjuncts in wound healing and like devices. Other embodiment compositions may be useful in areas of biomedical research, such as radio- and fluorescent-labelling of hyaluronic acid.

## Detailed Description of The Preferred Embodiments of The Invention

Hyaluronic acid is a well known and naturally occurring, water-soluble polysaccharide composed of repeating disaccharide units of glucuronic acid and N-acatylglucosamine. The disaccharide unit of hyaluronic acid or a salt thereof may be represented by the schematic formula: -

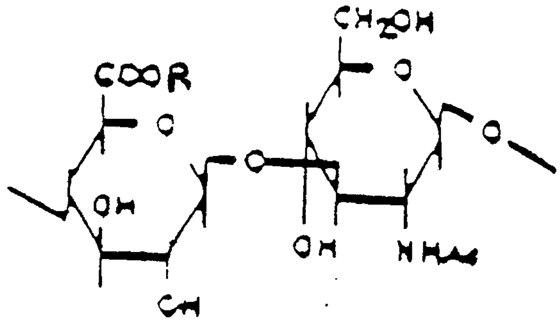

(I)

wherein Ac represents acetate and R represents hydrogen (in the case of the acid) or the cation of a salt (in the case of a salt). Preferably, the cation is an alkali metal cation, most preferably sodium ion. For convenience and brevity, the formula (I) given above will be referred to at times hereinafter by the equivalent formula

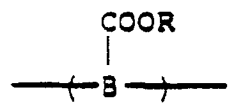

(II)

wherein R is as defined above and "B" has the obvious meaning ascribed to it. The polymer is hydrophilic.

Methods of preparing commercially available hyaluronic acid and salts thereof (particularly the physiologically acceptable salts such as the sodium salt) are well known.

For use in preparing the N-acylurea and O-acylisourea compounds of the invention, any of the known hyaluronic acid compositions, or salts thereof, as well as oligosaccharides or products obtained by physical, chemical or enzymatic treatment of the hyaluronic acid may be employed. These starting polymers will generally have "n" number of the units of formula (I) such that the polymer will have a weight average molecular weight ($M_w$) of from about 100,000 to about 12,000,000 Daltons (determined by calculation from the limiting viscosity number using the equation of Laurent et al., Biochimica et Biophysics Acta., 42: 476-485 [1960]).

The N-acylurea and O-acylisourea compounds of the invention may be prepared by reaction of hyaluronic acid or a salt thereof with a carbodiimide or a biscarbodiimide, in the presence of an available proton. The reaction conditions are well known (see Tetrahedron Report R101, supra.) and comprise protonation of the carbodiimide or biscarbodiimide in a first step. The acid anion attaches to the carbon atom of the cation formed, resulting in the formation of an O-acylisourea intermediate. The acyl group in the latter migrates from the oxygen atom to a nitrogen atom to give the N-acylurea derivative of the hyaluronic acid or salt. Generally the O --- N migration is incomplete, resulting in a product reaction mixture of both the N-acylurea and the O-acylisourea. The mixed products may be used separately or together to prepare the pharmaceutically active drug compositions of the invention.

The reaction may be schematically represented by the very simple formulae:

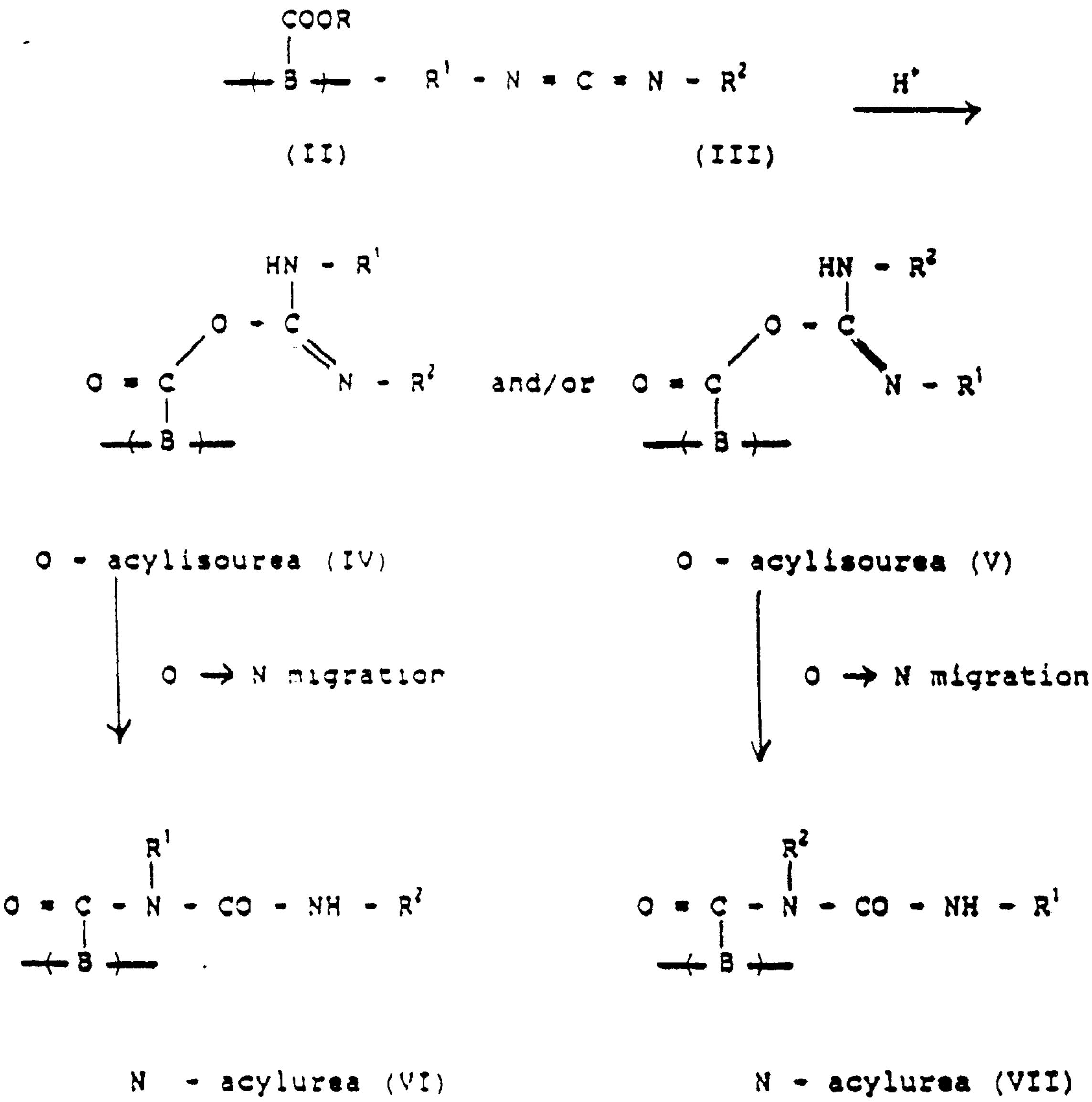


(II) (III)

and/or

O - acylisourea (IV)   O - acylisourea (V)

N - acylurea (VI)   N - acylurea (VII)

wherein R and B have the same meanings previously ascribed to them; the compound (III) is representative of a carbodiimide compound and $R^1$ and $R^2$ are each independently selected from the group consisting of hydrogen, hydrocarbyl, substituted- hydrocarbyl, alkoxy, aryloxy, alkaryloxy and the like.

The term "hydrocarbyl" as used herein means the monovalent moiety obtained upon removal of a hydrogen atom from a parent hydrocarbon. Representative of hydrocarbyl are alkyl of 1 to 25 carbon atoms, inclusive, such as methyl, ethyl, propyl, butyl, pentyl, hexyl, heptyl, octyl, nonyl, undecyl, decyl, dodecyl, octadecyl, nonodecyl, eicosyl, heneicosyl, docosyl, tricosyl, tetracosyl, pentacosyl and the isomeric forms thereof; aryl of 6 to 25 carbon atoms, inclusive, such as phenyl, tolyl, xylyl, napthyl, biphenyl, tetraphenyl and the like; aralkyl of 7 to 25 carbon atoms, inclusive, such as benzyl, phenethyl, phenpropyl, phenbutyl, phenhexyl, napthoctyl and the like; cycloalkyl of 3 to 8 carbon atoms, inclusive, such as cyclopropyl, cyclobutyl, cyclopentyl, cyclohexyl, cycloheptyl, cyclooctyl and the like; alkenyl of 2 to 25 carbon atoms, inclusive, such as vinyl, allyl, butenyl, pentenyl, hexenyl, octenyl, nonenyl, decenyl, undececyl, dodecenyl, tridecenyl, pentadecenyl, octadecenyl, pentacosynyl and isomeric forms thereof. Preferably, hydrocarbyl has 6 to 14 carbon atoms, inclusive.

The term "substituted hydrocarbyl" as used herein means the hydrocarbyl moiety as previously defined wherein one or more hydrogen atoms have been replaced with a chemical group which does not adversely affect the desired preparation of the product derivative. Representative of such groups are amino-phosphino-, quaternary nitrogen (ammonium), quaternary phosphorus (phosphonium), hydroxyl, amide, alkoxy, mercapto, nitro, alkyl, halo, sulfone, sulfoxide, phosphate, phosphite, carboxylate, carbamate groups and the like. Preferred substitute groups are amino, amide, ester and ammonium groups.

Preferred N-acylureas and O-acylisoureas of the invention are those in which $R^1$ and/or $R^2$ are

hydrocarbyl substituted with an amino group.

The term "alkoxy" as used herein means a monovalent group of the formula:

-O-alkyl

wherein the alkyl preferably has 4 to 12 carbon atoms, inclusive.

The term "aryloxy" as used herein means the monovalent group of the formula:-

-O-aryl

wherein the aryl preferably has 6 to 10 carbon atoms, inclusive and may be substituted as described above.

The term "alkaryloxy" as used herein means the monovalent group of formula:-

-O-alkylenearyl

such as oxybenzyl and the like.

The schematic formulae given above to represent the preparation of the N-acylureas and O-acylisoureas of the invention does not illustrate the reaction or products where a biscarbodiimide is used in place of the compound of formula (III), but that reaction scheme will suggest itself to the skilled artisan.

The carbodiimides and biscarbodiimides used to prepare the N-acylurea and O-acylisourea compositions of the invention are well known compounds, as represented by the formula (III) given above. Carbodiimides having the formula (III) are preferred where $R^1$ and/or $R^2$ represent more specifically alkyl, cycloalkyl, aryl or substituted forms thereof. Representative of a preferred class of monofunctional carbodiimides of formula (III) are

N-methyl-N′-tert-butylcarbodiimide
N,N′-diisopropylcarbodiimide
N,N′-dicyclohexylcarbodiimide
N,N′-ditert-butylcarbodiimide
N-cyclohexyl-N′-tert-butylcarbodiimide
N,N′-dibutylcarbodiimide
N,N′-diisobutylcarbodiimide
N-allyl-N′-propylcarbodiimide
N,N′-diallylcarbodiimide
N,allyl-N′-cyclohexylcarbodiimide
N-crotyl-N′-cyclohexylcarbodiimide
N-ally;-N′-(B-hydroxyethyl)carbodiimide
N-methyl-N′-propylcarbodiimide
N-propyl-N′tert-butylcarbodiimide
N-isopropyl-N′-tert-butylcarbodiimide
N-(α-dimethylaminopropyl)-N′tert-butylcarbodiimide
N-(β-bromoallyl)-N′-propylcarbodiimide
N-(β-bromoallyl)-N′-isopropylcarbodiimide
N-(β-bromoallyl)-N′-tert-butylcarbodiimide
N-(α-dimethylaminopropyl)-N′-(β-bromoallyl)carbodiimide
1-ethyl-3-(6-benzyloxylcarbonylaminohexyl)carbodiimide
1-(3-dimethylaminopropyl)-3-(6-benzoylaminohexyl)-carbodiimide
and the like.

The biscarbodiimides may be represented by those difunctional compounds of formula:-

$R^1\text{-}N=C=N\text{-}R^2\text{-}N=C=N\text{-}R^3$ (VIII)

wherein $R^1$ and $R^2$ have the same meanings given to them above and $R^3$ may have the same meaning as $R^1$.

Methods of preparing carbodiimides of the formula (III) and biscarbodiimides of the formula (VIII) are well known and need not be recited herein in detail; see for example the methods described in the U.S. Patents 2,946,819; 3,231,610; 3,502,722; 3,644,456; 3,972,933; 4,014,935; 4,066,629: 4,085,140: 4,096,334: and 4,137,386, all of which are incorporated herein by reference thereto.

By appropriate selection of a particular carbodiimide or biscarbodiimide or a class of such compounds, the physical properties of the N-acylurea and O-acylisourea compounds of the invention may be tailored for advantageous use in particular applications. For example, hydrophobic and/or cationic "side-arms" may be attached to the hyaluronic polymer, to prepare useful polymer carriers for therapeutic drugs by selecting monofunctional carbodiimides. So also, lipophilic side-arms may be attached to the hyaluronic acid polymer, by selection of the $R^1$ and $R^2$ moieties.

More importantly, carbodiimide originated side-arms are covalently attached to the hyaluronic acid polymer chain as N-acylurea or O-acylisourea side-arms. Free functional groups in the side-arm (amine, amide, ester) can be further reacted to bond with reactive therapeutic drug molecules, to obtain vehicles for

delivery of therapeutic drugs, under conventional and known reaction conditions.

In carrying out the preparation of the N-acylurea and O-acylisourea compounds of the invention, a sufficient proportion of the carbodiimide is reacted with the hyaluronic acid or salt thereof to obtain a polymer chain having recurring polymer chain units of the formula (I) given above, interrupted by at least one disaccharide unit per hyaluronic acid molecule having a pendant N-acylurea or O-acylisourea side-arm, for example a chain unit of the formula (IV), (V), (VI) or (VII) given above. In preferred compositions of the invention, from about 0.5 to about 30 percent of the original chain units of the formula (I) are converted to chain units like those of the formula (IV), (V), (VI) or (VII). Generally an excess of the stoichometric proportion of carbodiimide is advantageous to promote the desired reaction.

Use of a biscarbodiimide reactant to prepare N-acylureas and O-acylisoureas of the invention results in a cross-linking between adjacent hyaluronic acid molecules, since the biscarbodiimide is difunctional. In this case, the "pendant side-arm" referred to above is shared by two molecules of hyaluronic acid. These cross-linked materials are particularly useful as biomaterials and as relatively insoluble matrices for drug delivery since the biscarbodiimide cross-linked hyaluronic acid possesses new drug binding regions on the cross-link, which does not interfere with biocompatibility.

The biscarbodiimide cross-linked hyaluronic acid is found to be a hydrogel. The term "hydrogel" is defined herein to mean a cross-linked macromolecular network swollen in water or biological fluids. The degree of gelation is of course dependent on the degree of cross-linking achieved.

The reaction conditions for hyaluronic acid cross-linking with a biscarbodiimide are similar to those used for hyaluronic acid-monocarbodiimide coupling reactions. Advantageously, the cross-linking reactions are carried out with (1) an increase of the hyaluronic acid concentration in the reaction mixture, and (2) a decrease of the biscarbodiimide concentration in the addition solution. This creates a condition favorable to intermolecular cross-linking versus intramolecular cross-linking.

The reactions described above may be directed to favor the formation of the N-acylurea derivatives; i.e.; compounds of formula (VI) or (VII) by increasing the pH with aqueous base or by the addition of amine bases in varying amounts. Representative of amine bases are primary, secondary and tertiary amines and diamines such as for example methylamine, ethylamine, 1,6-diaminohexane and the like. Amine promoted O → N migration is described by Mikolajczyk et al., Tetrahedron, 37, pg. 251 (1981).

The preparative reaction may be carried out in the presence of an inert solvent, i.e., a solvent which does not enter into or otherwise adversely affect the desired course of the reaction. Representative of such solvents are water, alkanols, dimethylformamide, dimethylsulfoxide and the like. Sources of protons to carry out the preparative reaction may include dilute mineral acids such as, for example, hydrochloric acid.

The reaction proceeds satisfactorily over a wide range of temperatures, for example from -20° C. to 80° C., preferably at room temperatures.

Progress of the reaction described above may be followed by measurement of pH. When the pH is stabilized, the reaction is substantially complete. At the conclusion of the reaction, the desired N-acylurea and O-acylisourea derivative may be separated from the reaction mixture by conventional methods, for example by precipitation, washing and re-precipitation. The completeness of the reaction, the nature of the products and the extent of chemical modification can be determined by proton NMR on solutions of the modified hyaluronic acid solubilized in dilute NaOD to reduce viscosity.

The following examples and preparations describe the manner and process of making and using the invention and set forth the best mode contemplated by the inventors of carrying out the invention but are not to be construed as limiting the invention. Where reported, the following tests were carried out.

Intrinsic Viscosity (IV) :

The intrinsic viscosity was measured using a Cannon-Ubbelohde semi-micro dilution viscometer, size 75, at 37° C. and is reported in milliliters/gram (ml/g).

Dynamic Viscosity (DV):

The dynamic viscosity is determined by Brookfield digital viscometer, Model RVTOCP (with spindle No. 52), and is reported in centipoise (Cps) at a concentration of 10 mg/ml, shear rate of 1.0 sec.

Molecular Weight (Mw):

The weight average molecular weights ($M_w$) were calculated from the limiting viscosity number using the equation of Laurent et al., supra.

Preparation 1

$C_2H_5$ -N=C=N-$C_6H_{12}$ - NH - CO - O - $CH_2$ - $C_6H_6$

1-ethyl-3-(6-benzyloxycarbonylaminohexyl)carbodiimide

Mercuric oxide (100 mg) was suspended in 15 ml dry acetone, to which 1-ethyl-3-(6-benzyloxylcarbonylaminohexyl)2-thiourea (77 mg) dissolved in 5 ml of dry acetone was added. The reaction mixture was refluxed and the oil bath was kept at 80° C. After 20 minutes, the reaction mixture turned black and the reflux continued for three hours. The formed black mercuric sulfide stuck to the reaction flask and the solution was clear. The solution was then filtered through Celite, dried with magnesium sulfate and evaporated to give 60 mg of 1-ethyl-3-(6-benzyloxycarbonylaminohexyl) carbodiimide with a purity of 93% (GC).

Preparation 2

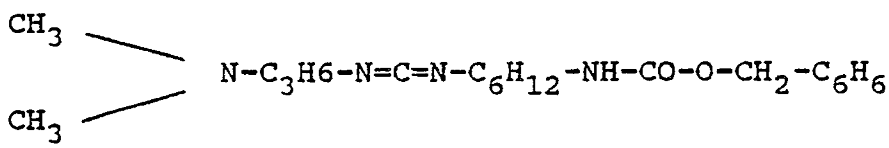

1-(3-dimethylaminopropyl)-3(6-benzoyloxylcarbonylaminohexyl) carbodiimide.

The same procedure of Preparation 1, supra., but refluxing 1-(3-dimethylaminopropyl),3-(6-benzyloxylcarbonylaminohexyl)2-thiourea in acetone in the presence of mercuric oxide was followed, except the crude product was dried with magnesium sulfate and chromatographed on silica gel (chloroform, triethylamine). Thus 77 mg of the thiourea gave 15 mg of 1-(3-dimethylaminopropyl)-3(6-benzyloxylcarbonyl-aminohexyl) carbodiimide.

Example 1

N-ACYLUREA FORMED BY THE REACTION OF SODIUM HYALURONATE WITH 1-(3-DIMETHYLAMINOPROPYL)-3-ETHYLCARBODIIMIDE IN THE PRESENCE OF EXCESS AMINE

Sodium hyaluronate (246 mg, 0.615 m mole equivalents) with 17.82 mg (0.307 m mole equivalent) of 1,6-diaminohexane dissolved in water (pH adjusted to 4.75) was mixed with 1-(3-dimethylaminopropyl)-3-ethylcarbodiimide (11.78 mg, 0.062 m mole) dissolved in water. The pH of the mixture was adjusted to 4.75 with 0.1N hydrochloric acid and maintained at that pH during the course of the reaction, with 0.1N hydrochloric acid. The mixture is stirred at room temperature for two hours. At the end of the period of time, sodium chloride was added to make a concentration of 5 percent (w/v). Ethanol equal to three volumes of the reaction mixture was then added, and a white stringy precipitate formed. The precipitate was separated, dried and redissolved in water which was then diluted for a second precipitation. The precipitation and redissolution sequence was repeated three times. The final solution of the N-acylurea product was clear and viscous. No O-acylisourea product was detected.

Example 2

ACYLUREA FORMED BY THE REACTION OF SODIUM HYALURONATE WITH 1-ETHYL-3-(6-BENZYLOXYLCARBONYLAMINOHEXYL)CARBODIIMIDE

Repeating the general procedure of Example 1 , supra. but without the presence of 1,6-diaminohexane and reacting 90.6 mg (0.225 m equivalent) of sodium hyaluronate with 18 mg (0.048 m mole) of 1-ethyl-3-(6-benzyloxylcarbonylaminohexyl-carbodiimide ( Preparation 1. supra. ) There was obtained a product N-acylurea (105 mg of lyophilized product). Compared to the starting sodium hyaluronate, the product was characterized in part by the following physical properties:

| | Product N-Acylurea | Starting Sodium Hyaluronate |
|---|---|---|
| Intrinsic Viscosity (ml/g) | 2734 | 2354 |
| Dynamic Viscosity (cps) | 24332 | 23199 |

The product represents sodium hyaluronate where 16.98 percent of the polymer chain units of formula (I) given above have been converted to the formula (II) where R is the reaction residue of the carbodiimide.

Example 3

Repeating the general procedure of Example 1, supra. but leaving out the 1,6-diaminohexane and replacing the 1-(3-dimethylaminopropyl)-3-ethylcarbodiimide as used therein with an equal proportion of 1-(3-dimethylaminopropyl)-3-(6-benzyoloxylcarbonylaminohexyl)carbodiimide prepared in accordance with Preparation 2, supra. there is obtained the corresponding N-acylurea having chain units of the formula (II) given above, wherein R is the reaction residue of the carbodiimide.

Example 4

ACYLUREA FORMED BY THE REACTION OF SODIUM HYALURONATE WITH 1-ETHYL-3-TRIDECYL-CARBODIIMIDE

The general procedure of Example 3 , supra., was repeated, except that 401 mg (1.0 m equivalent) of sodium hyaluronate was reacted with 25.2 mg (0.10 m mole) of 1-ethyl-3-tridecyl-carbodiimide to obtain 386 mg of the lyophilized acylurea (IV = 2591) having chain units of formula (II) given above wherein R is the reaction residue of the carbodiimide.

Example 5

N-ACYLUREA FORMED BY THE REACTION OF SODIUM HYALURONATE WITH 1-ETHYL-3-OCTYL-CARBODIIMIDE

Repeating the procedure of Example 3 supra., but replacing the 1-ethyl-3-tridecylcarbodiimide as used therein with 45.6 mg (0.238 m moles) of 1-ethyl-3-octylcarbodiimide and using 477 mg (1.119 m equivalent) of hyaluronate there was obtained 465 mg of the lyophilized acylurea product having chain units of formula (II) given above wherein R is the reaction residue of the carbodiimide.

The product exhibited an IV of 2534 ml/g.

Example 6

## N-ACYLUREA AND O-ACYLISOUREA FORMED BY THE REACTION OF SODIUM HYALURONATE WITH P-PHENYLENE-BIS(ETHYL) CARBODIIMIDE

Following the general procedure of Example 1. supra ., but without the added presence of the 1,6-diaminohexane, 404 mg (1.01 m equivalent) of sodium hyaluronate was reacted with 10.7 mg (0.05 m mole) of p-phenylenebis(ethyl)-carbodiimide to obtain 393 mg of the N-acylurea lyophilized product of formula:- B-CO-N$C_2H_5$ - CO - HN - $C_6H_6$ - NH - CO - N$C_2H_5$ -CO- B and the O-acylisourea lyophylized product of formula:

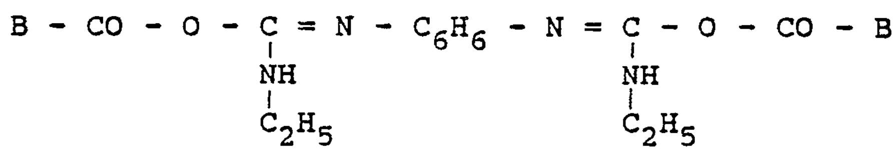

wherein B has the meaning previously ascribed to it. The ratio of N-acylurea to O-acylisourea is about 1:2.3.

The degree of crosslinking was calculated from the percentage ratio of the added carbodiimide group over the hyaluronate's carboxyl group, to be 18 percent. The IV of the 10% crosslinked hyaluronic acid was 3073 ml/g as compared with the control IV of 2354 ml/g. The 18% crosslinked product was not soluble in water, but swelled into a gel which was stable in water at room temperature.

Example 7

## N-ACYLUREA FORMED BY THE REACTION OF SODIUM HYALURONATE WITH 1.6-HEXYLENEBIS(ETHYL)-CARBODIIMIDE

Repeating the procedure of Example 6 , supra., but replacing the p-phenylenebis(ethyl)carbodiimide as used (therein with 11 mg (0.05 m. mole) of 1,6-hexylenebis(ethyl)carbodiimide, and using 416 mg (1.09m equivalent) of hyaluronate there was obtained 400 mg of the N-acylurea product of formula:

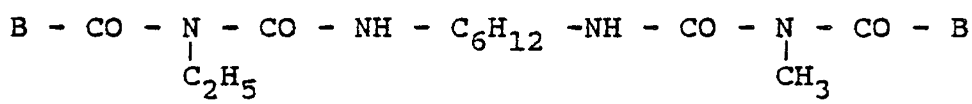

wherein B is as defined above. The product had an IV of 2554 ml/g. The degree of cross-linking was calculated to be 10 percent. Placement in water produced a viscous solution. No O-acylisourea was detected.

Example 8

## THE N-ACYLUREA AND THE O-ACYLISOUREA OF SODIUM HYALURONATE AND 1-ETHYL-3-(6-TRIFLUOROACETAMIDO)HEXYL-CARBODIIMIDE

Following the general procedure of Example 6 , supra., 400 mg (1.0 m equivalent) of sodium hyaluronate and 60 mg. (0.22 m equivalent) of 1-ethyl-3-(tri-fluoroacetamido) hexyl-carbodiimide dissolved in 5 ml of isopropanol was reacted to obtain 392 mg (85.2% of theory) of N-acylurea of formula:

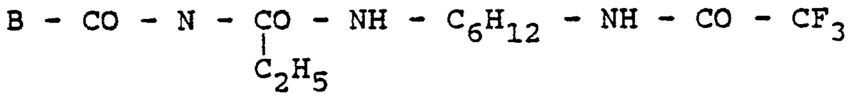

and O-acylisourea of formula:

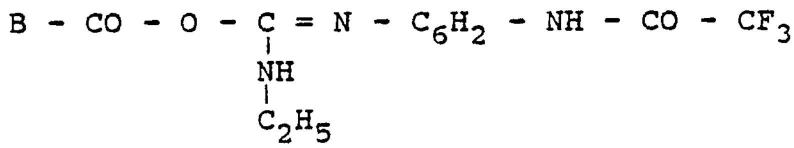

wherein B has the meaning previously given to it. The ratio of N-acylurea to O-acylisourea is about 85:15. Upon basic hydrolysis, the trifluoroacetyl group will be removed and the free amine group produced for reaction with a therapeutic drug, preferably by covalently bonding. For example, the amine group can form a covalent bond with the carboxyl group associated with the therapeutic drug naprosyn. Naprosyn and other drugs will also bind to the N-acylureas and O-acylisoureas of the invention which have hydrophobic regions by a second mechanism, i.e.; hydrophobic interaction.

From the examples given above it will be appreciated that the modification of the hyaluronic acid by reaction with a carbodiimide or biscarbodiimide does not adversely degrade the polymer. The properties of viscoelasticity are retained. Unexpectedly, the intrinsic viscosity is increased in the derivative compound. This may be advantageous in some cases where a high viscosity is desired for implanting a therapeutic drug.

## Claims

1. A compound selected from the group consisting of the N-acylurea and O-acylisourea derivative of hyaluronic acid or a salt thereof; said derivative being free of chemically bound collagen.

2. The compound of claim 1 wherein the hyaluronic acid has a weight average molecular weight of from about 100,000 to about 12,000,000 Daltons determined by calculation from the limiting viscosity number.

3. The compound of claim 1 prepared by reaction of the acid or salt with a member selected from the class consisting of a carbodiimide and a biscarbodiimide.

4. The compound of claim 3 wherein the member is a carbodiimide selected from those of the formula:
$R^1$-N=C=N-$R^2$
wherein $R^1$ and $R^2$ are each independently selected from the group consisting of hydrogen, hydrocarbyl, substituted-hydrocarbyl, alkoxy, aryloxy and alkaryloxy.

5. The compound of claim 4 wherein at least one of $R^1$ and $R^2$ is an amino-substituted hydrocarbyl.

6. The compound of claim 5 wherein the member is 1-ethyl-3-(6-benzyloxycarbonylaminohexyl)-carbodiimide.

7. The compound of claim 5 wherein the member is 1-(3-dimethylaminopropyl)-3(6-benzoyloxyl- carbonylaminohexyl)carbodiimide.

8. The compound of claim 5 wherein the member is 1-(3-dimethylaminopropyl-3-ethylcarbodiimide.

9. The compund of claim 1 which is the reaction product of 1-ethyl-3-tridecyl-carbodiimide and hyaluronic acid or a salt thereof.

10. The compound of claim 1 which is the reaction product of 1-ethyl-3-octyl carbodiimide and hyaluronic acid or a salt thereof.

11. The compound of claim 3 wherein the member is a biscarbodiimide selected from those of the formula:
$R^1$ - N = C = N - $R^2$ - N = C = N - $R^3$
wherein $R^1$ amd $R^2$ have the same meanings ascribed to them in claim 4 and $R^3$ has the meaning given $R^1$.

12. The compound of claim 11 wherein the member is p-phenylenebis(ethyl)carbodiimide.

13. The compound of claim 11 wherein the member is 1,6-hexylenebis(ethyl)carbodiimide.

14. The N-acylurea of claim 1.

15. The O-acylisourea of claim 1.

16. A device which comprises a water-insoluble N-acylurea or an O-acylisourea derivative of hyaluronic acid and a biscarbodiimide.

17. A water-soluble N-acylurea or O-acylisourea derivative of hyaluronic acid and a carbodiimide.